(19) Europäisches Patentamt
European Patent Office
Office européen des brevets

(11) Numéro de publication: **0 071 708**
**B1**

# (12) FASCICULE DE BREVET EUROPEEN

(45) Date de publication du fascicule du brevet: **11.07.84**

(51) Int. Cl.³: **C 07 C 49/24**, C 07 C 45/00

(21) Numéro de dépôt: **82104702.4**

(22) Date de dépôt: **28.05.82**

(54) **Procédé pour la préparation d'hydroxy-cétones.**

(30) Priorité: **24.07.81 CH 4842/81**

(43) Date de publication de la demande:
**16.02.83 Bulletin 83/7**

(45) Mention de la délivrance du brevet:
**11.07.84 Bulletin 84/28**

(84) Etats contractants désignés:
**CH DE FR GB LI**

(56) Documents cités:
**EP - A - 0 021 769**
**DE - A - 2 305 140**

**Patent Abstracts of Japan Vol. 4, no. 93, 5 juillet 1980, page 21C17**
**Chemical Abstracts vol. 95, no. 7 17 août 1981, Columbus, Ohio, USA H. ISHIKAWA et al. "Reaction of acetals with Grignard reagents" page 651, colonne 2 à page 652, colonne 1, abstract no. 61392n**

(73) Titulaire: **FIRMENICH SA, 1, route des Jeunes, CH-1211 Geneve 8 (CH)**

(72) Inventeur: **Schulte-Elte, Karl-Heinrich, 44, chemin de Carabot, CH-1213 Onex (CH)**
Inventeur: **Snowden, Roger, 5, chemin des Palettes, CH-1212 Grand-Lancy (CH)**
Inventeur: **Müller, Bernard, 16, route de Malagnon, CH-1208 Genève (CH)**

(74) Mandataire: **Salvadori, Giuseppe, Dr., c/o Firmenich S.A. Case Postale 239, CH-1211 Genève 8 (CH)**

**EP 0 071 708 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

Le 2-[2-méthyl-prop-1-ène-1-yl]-4-méthyl-tétrahydropyranne, un éther terpénique de formule

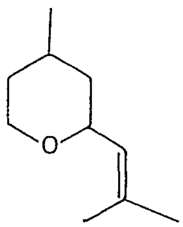

mieux connu sous la dénomination d'oxyde de rose, est un composé fort apprécié dans l'industrie de la parfumerie.

Depuis sa découverte en 1960 [voir Brevet Suisse No. 395 406], nombreuses ont été les publications scientifiques relatant des synthèses nouvelles pour sa préparation.

La plupart des méthodes connues prévoient l'utilisation d'intermédiaires acycliques ou l'introduction du substituant 2-isobutényle sur l'anneau pyrannique préalablement formé. Une approche synthétique différente, à partir de 3-méthyl-but-2-ène-1-al et 2-méthyl-but-1-ène-4-ol, avait été formulée par J.P.H. Tyman et B.J. Willis [voir Tetrahedron Letters, 51, 4507 (1970)], ladite méthode pouvant être illustrée comme suit:

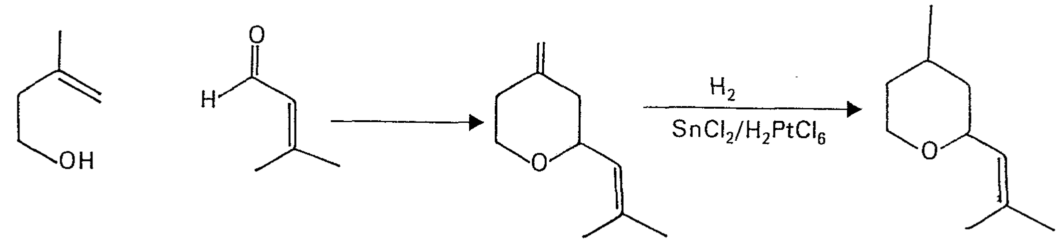


Suivant les auteurs cités ci-dessus, la réduction de l'intermédiaire méthylénique, ou déhydroxyde de rose, peut être effectuée par hydrogénation en phase homogène en présence de chlorure d'étain II et d'acide hexachloroplatinique. L'on pouvait ainsi obtenir un mélange constitué par environ 91% de l'isomère cis et 9% de l'isomère trans.

La demande de brevet internationale WO 79/00509 fait également état d'un procédé destiné à préparer tout particulièrement l'oxyde de rose sous forme d'un mélange isomérique contenant des quantités prépondérantes d'isomère cis, ledit procédé utilisant, tout comme Tyman et Willis, le 3-méthylbut-2-ène-1-al et le 2-méthyl-but-1-en-4-ol à titre de produits de départ, mais faisant intervenir une étape d'isomérisation à l'aide d'un agent acide.

L'intérêt présenté par l'oxyde de rose représente en soi une source de stimulation à rechercher des voies alternatives pour sa fabrication industrielle.

La présente invention apporte précisément une solution nouvelle à ce problème. Elle a en effet trait à un procédé pour la préparation d'hydroxycétones de formule

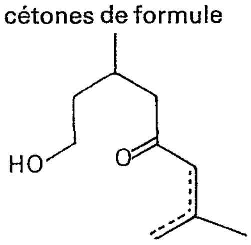

(Ia, b)

possédant une double liaison dans l'une des positions indiquées par les pointillés, ledit procédé étant caractérisé en ce qu'on traite au moyen d'une base forte, et dans un solvant organique inerte, un carbinol diallylique de formule

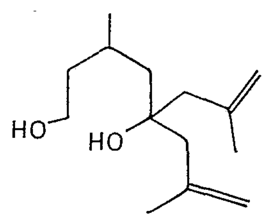

(II)

Les composés de formule (I) sont des produits intermédiaires utiles à la préparation d'oxyde de rose, comme illustré par le schéma suivant:

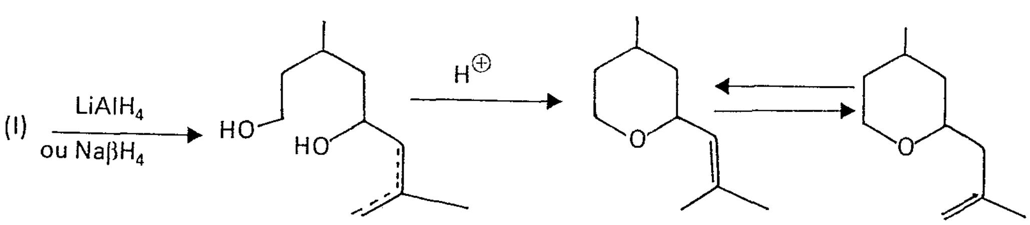


* [voir Synthesis 27 (1980) et Bull. Chem. Soc. Japan 54, 776 (1981)]

Ledit carbinol diallylique (II) peut être obtenu au moyen d'une réaction de type de Grignard à partir de la 3-méthyl-valérolactone et du magnésien d'un halogénure de méthallyle suivant le schéma que voici:

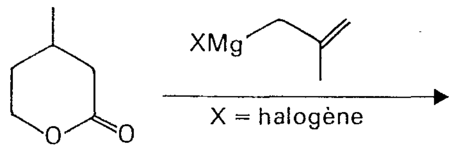


(II)

La réaction, qui caractérise le procédé de l'invention, consiste formellement en un clivage anionique, lequel est effectué par l'action d'une base forte. A cet effet, on peut utiliser des bases minérales ou organiques telles que les hydrures, les alkoxydes ou les hydroxydes d'un métal alcalin, le sodium et le potassium de préférence. Parmi lesdites bases, il convient de citer tout particulièrement l'hydrure de sodium ou potassium, le tert-butylate de sodium ou potassium, le tert-amylate de sodium ou le méthylate et l'éthylate de sodium.

Le choix particulier, parmi les bases appartenant à la classe définie ci-dessus, est déterminé par des considérations d'ordre économique, de sécurité et d'hygiène du travail. C'est ainsi que les alkoxydes sont préférés aux hydrures, le tert-butylate de potassium ou sodium étant employé de préférence.

On a pu déterminer que la proportion de la base utilisée doit être égale ou supérieure à la quantité stœchïométrique requise. En réalité, les rendements les meilleurs ont été obtenus en utilisant la base en excès.

Les temps de réaction observés sont relativement courts.

Bien entendu, la température exerce une influence déterminante sur les temps de réaction. Le procédé, exothermique en soi, peut s'effectuer à une température voisine de la température ambiante. Des températures comprises entre 20° et 90°C sont employées de préférence à des températures inférieures les temps de réaction devenant trop longs.

Des températures dépassant la limite supérieure indiquée peuvent être employées avec succès, notamment en effectuant la réaction dans des solvants aprotiques, par exemple dans la méthyl-pyrrolidone.

Comme indiqué plus haut, la réaction s'effectue dans un solvant organique inerte. A cet effet, on peut utiliser des solvants organiques tels des éthers, comme le tétrahydrofuranne ou l'éther diisopropylique, des amides telles la diméthylformamide ou l'hexaméthyl-triamidure de phosphore, un hydrocarbure aromatique, le benzène ou toluène par exemple, un alcool comme l'alcool éthylique ou le tert-butanol ou encore la méthylpyrrolidone ou le diméthylsulfoxyde. Des mélanges desdits solvants peuvent également être employés. Selon un mode d'exécution particulier, on utilise comme base le tert-butylate de potassium et en tant que solvant, la diméthylformamide ou un mélange de diméthylformamide et tétrahydrofuranne.

Les hydroxy-cétones de formule (Ia, b) obtenues grâce au procédé de l'invention sont, dans les conditions de réaction employées, en équilibre avec les hémiacétals (Ic, d).

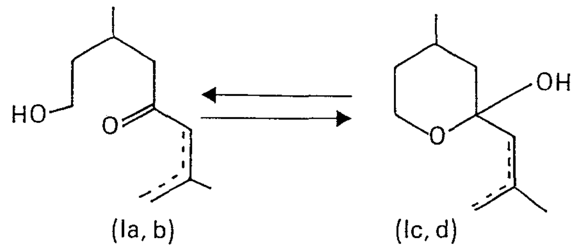

(Ia, b) (Ic, d)

Nous avons en effet observé que par le procédé de l'invention on obtenait des mélanges contenant des quantités variables des quatre composés, à savoir le 3,7-diméthyl-5-oxo-oct-6-énol, le 3,7-diméthyl-5-oxo-oct-7-énol, le tétrahydro-4-méthyl-2-(2-méthyl-2-propényl)-2- (2H)-pyranol et le tétrahydro-4-méthyl-2-(2-méthyl-1-propényl)-2(2H-pyranol, le 3,7-diméthyl-5-oxo-oct-6-énol étant formé en quantité prépondérante, de l'ordre de 80%.

L'invention est illustrée en détail par l'exemple suivant, dans lequel les températures sont indiquées en degrés centigrades.

**Exemple**

22,6 g (0,1 M) de 3,7-diméthyl-5-hydroxy-5-(2-méthyl-2-propényl)-oct-7-énol et 22 g (0,2 M) de tert-butylate de potassium dans 100 ml de diméthylformamide (DMF) ont été chauffés à 80°, sous atmosphère d'azote et agitation, pendant 3 heures. Après refroidissement, le mélange de réaction a été versé avec agitation dans une solution d'acide acétique glaciale (22 g) dans 100 g de glace, puis il a été repris avec 200 ml d'éther de pétrole. Les extraits organiques réunis ont été lavés avec successivement de l'acide chlorhydrique, de l'eau et une solution diluée de NaOH jusqu'à neutralité.

L'évaporation de la phase organique a fourni 17 g d'un résidu constitué par environ 85% de 3,7-diméthyl-5-oxo-oct-6-énol et 3,7-diméthyl-5-oxo-oct-7-énol, et environ 15% des hémiacétals des formules (Ic) et (Id).

IR: 3400, 3080, 1715, 1675, 1640, 1620, 1050–1100 et 895 $cm^{-1}$; RMN (60 MHz): 0,85–1,05; 1,72 et 1,18; 3,4–4,2; 4,8 et 4,95 δ ppm.

**Revendications**

1. Procédé pour la préparation d'hydroxy-cétones de formule

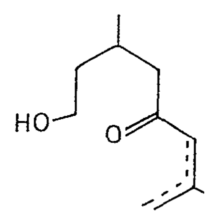

(Ia, b)

possédant une double liaison dans l'une des positions indiquées par les pointillés, caractérisé en

ce qu'on traite au moyen d'une base forte, et dans un solvant organique inerte, un carbinol diallylique de formule

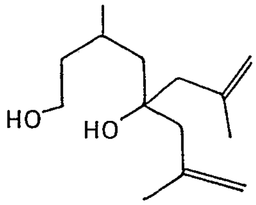

(II)

2. Procédé suivant la revendication 1, caractérisé en ce qu'on utilise comme base forte un hydrure, un alkoxyde ou un hydroxyde d'un métal alcalin.

3. Procédé suivant la revendication 2, caractérisé en ce qu'on utilise comme alkoxyde le tert-butylate de potassium.

4. Procédé suivant la revendication 3, caractérisé en ce que la réaction est effectuée dans la diméthylformamide à une température comprise entre 20 et 90°C.

## Claims

1. Process for the preparation of hydroxy-ketones of formula

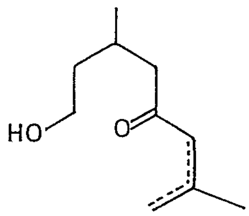

(Ia, b)

having a double bond in one of the positions indicated by the dotted lines, characterized in that a diallyl carbinol of formula

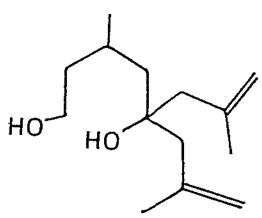

(II)

is treated with a strong base in an inert organic solvent.

2. Process according to claim 1, characterized in that the strong base is an alkali metal hydride, alkoxide or hydroxide.

3. Process according to claim 2, characterized in that the alkoxide is potassium tert-butoxide.

4. Process according to claim 3, characterized in that the reaction is effected in dimethylformamide at a temperature of between 20° and 90°C.

## Patentansprüche

1. Verfahren zur Herstellung von Hydroxy-ketonen der Formel

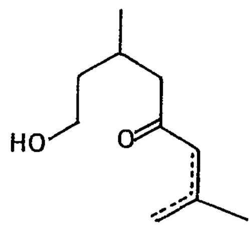

(Ia, b)

welche in einer der durch die gestrichelten Linien gekennzeichneten Stellungen eine Doppelbindung besitzt, dadurch gekennzeichnet, dass man ein Diallylcarbinol der Formel

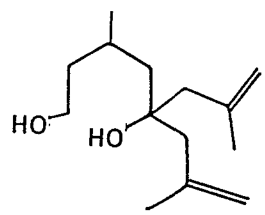

(II)

mit einer starken Base in einem inerten organischen Lösungsmittel umsetzt.

2. Verfahren gemäss Anspruch 1, dadurch gekennzeichnet, dass die starke Base ein Alkalimetall-Hydrid, -Alkoxyd oder -Hydroxyd ist.

3. Verfahren gemäss Anspruch 2, dadurch gekennzeichnet, dass das Alkoxyd Kalium tert-Butoxyd ist.

4. Verfahren gemäss Anspruch 3, dadurch gekennzeichnet, dass die Reaktion in Dimethylformamid bei einer Temperatur zwischen 20° und 90°C durchgeführt wird.